(19) **Europäisches Patentamt / European Patent Office / Office européen des brevets**

(11) **EP 4 314 762 B1**

(12) **EUROPÄISCHE PATENTSCHRIFT**

(45) Veröffentlichungstag und Bekanntmachung des Hinweises auf die Patenterteilung:
**28.05.2025 Patentblatt 2025/22**

(21) Anmeldenummer: **22706850.9**

(22) Anmeldetag: **21.02.2022**

(51) Internationale Patentklassifikation (IPC):
**G01N 1/38** *(2006.01)*  **G01N 1/20** *(2006.01)*
**G01N 33/18** *(2006.01)*  **G01N 9/00** *(2006.01)*
**G01N 9/36** *(2006.01)*

(52) Gemeinsame Patentklassifikation (CPC):
**G01N 1/38; G01N 1/2035; G01N 9/002; G01N 9/36; G01N 33/18;** G01N 2001/383

(86) Internationale Anmeldenummer:
**PCT/EP2022/054261**

(87) Internationale Veröffentlichungsnummer:
**WO 2022/199961 (29.09.2022 Gazette 2022/39)**

(54) **VERFAHREN ZUM BESTIMMEN EINES PROBENSTROMKONZENTRATIONSWERTS EINES ANALYTEN IN EINEM PROBENSTROM**

**METHOD TO DETERMINE FLOWING SAMPLE CONCENTRATION VALUE OF AN ANALYTE IN A SAMPLE FLOW**

**MÉTHODE POUR LA DÉTERMINATION DE LA CONCENTRATION D'UN ANALYTE DANS UN ÉCHANTILLON EN ÉCOULEMENT**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.03.2021 DE 102021107684**

(43) Veröffentlichungstag der Anmeldung:
**07.02.2024 Patentblatt 2024/06**

(73) Patentinhaber: **TrueDyne Sensors AG
4153 Reinach (CH)**

(72) Erfinder:
• **HUBER, Christof
3007 Bern (CH)**
• **RITTER, Josua
4153 Reinach (CH)**
• **VON MÖLLENDORFF, Ragnar
79639 Grenzach-Wyhlen (DE)**
• **BAUER, Johannes
79540 Lörrach (DE)**

(74) Vertreter: **Endress + Hauser Group Services
(Deutschland) AG+Co. KG
Colmarer Straße 6
79576 Weil am Rhein (DE)**

(56) Entgegenhaltungen:
DE-A1- 102019 123 874  US-A- 5 423 228
US-A1- 2011 077 144  US-A1- 2021 080 446
US-B2- 10 684 153

**Beschreibung**

Die vorliegende Erfindung betrifft ein Verfahren zum kontinuierlichen Bestimmen eines Probenstromkonzentrationswerts eines Analyten in einem Probenstrom, welcher einen Analyten in einem Lösungsmittel enthält.

Insbesondere dann, wenn der Analyt eine Konzentration nahe der Sättigungskonzentration aufweist, kann er aus dem Lösungsmittel ausscheiden und sich an Oberflächen von Messgeräten ablagern, so dass die Konzentrationsmessung beeinträchtigt wird. Dies ist beispielsweise bei hochkonzentrierten Solen, beispielsweise Kochsalzlösungen der Fall, für die grundsätzlich eine Dichtemessung mit vibronischen Sensoren zur Konzentrationsbestimmung geeignet ist. Wenn jedoch Kochsalz an den Sensoroberflächen abgeschieden wird, dann wird die Dichtemessung und damit Konzentrationsbestimmung verfälscht.

Es ist daher die Aufgabe der vorliegenden Erfindung, hier Abhilfe zu schaffen.

Die Aufgabe wird erfindungsgemäß gelöst durch das Verfahren gemäß Patentanspruch 1.

Das erfindungsgemäße Verfahren dient zum, insbesondere kontinuierlichen Bestimmen eines Probenstromkonzentrationswerts eines Analyten in einem Probenstrom, welcher den Analyten und ein Lösungsmittel aufweist, wobei das Verfahren folgende Schritte umfasst:

> Präparieren eines Messstromes durch Verdünnen des Probenstromes mit einem Lösungsmittelstrom;
> Ermitteln eines Messstromzentrationswerts des Analyten in dem Messstrom; und
> Ermitteln des Probenstromkonzentrationswerts des Analyten in dem Probenstrom, in Abhängigkeit des Messstromkonzentrationswerts und eines Verdünnungsverhältnisses von Probenstrom und Messstrom.

In einer Weiterbildung der Erfindung umfasst das das Ermitteln des Messstromkonzentrationswert das Erfassen einer Messgröße, welche von der Konzentration des Messstroms in dem Analyten abhängt. In einer Ausgestaltung dieser Weiterbildung der Erfindung umfasst die Messgröße die Dichte des Messstroms.

In einer Weiterbildung der Erfindung wird das Verdünnungsverhältnis auf Basis eines Massedurchflussratenmesswerts des Lösungsmittelstroms und eines Massedurchflussratenmesswerts des Messstroms ermittelt.

In einer Weiterbildung der Erfindung wird das Verdünnungsverhältnis auf einen konstanten Wert geregelt.

In einer Weiterbildung der Erfindung wird das Verdünnungsverhältnis geregelt, indem der Massdurchflussratenmesswert des Lösungsmittelstroms auf ein konstantes Verhältnis zum Massendurchflussratenmesswert des Messstroms geregelt wird, wobei das Verhältnis nicht mehr 1/2, beispielsweise nicht mehr als 1/4, und insbesondere nicht mehr als 1/5 beträgt.

In einer Weiterbildung der Erfindung umfasst der Analyt ein Salz, insbesondere NaCl.

In einer Weiterbildung der Erfindung umfasst das Lösungsmittel ein wässriges Medium, insbesondere Wasser.

In einer Weiterbildung der Erfindung wird ein Dichtemesswert für die Dichte des Messstroms mittels eines vibronischen auf Basis mindestens einer Schwingfrequenz des vibronischen Sensors ermittelt.

In einer Weiterbildung der Erfindung wird der Messstrom nach Erfassen der Messgröße und bestimmen des Massendurchflussratenmesswerts des Messstroms verworfen.

In einer Weiterbildung der Erfindung wird der Probenstrom einem Prozessmedienstrom entnommen, dessen Massedurchflussrate nicht weniger als das Hundertfache insbesondere nicht weniger als das Tausendfache der Massedurchflussrate des Probenstroms beträgt, wobei der Probenstromkonzentrationswert als Prozessmedienkonzentrationswert ausgegeben wird.

In der Erfindung weist der Probenstrom eine Konzentration des Analyten auf, welche eine Sättigungskonzentration des Analyten in dem Lösungsmittel um nicht mehr als 4 % der Sättigungskonzentration und insbesondere nicht mehr als 2 % der Sättigungskonzentration unterschreitet.

In der Erfindung weist der Messstrom eine Konzentration des Analyten auf, welche eine Sättigungskonzentration des Analyten in dem Lösungsmittel um nicht weniger als 8 % der Sättigungskonzentration und insbesondere nicht weniger als 12 % der Sättigungskonzentration unterschreitet.

Eine Vorrichtung zum Bestimmen eines Probenstromkonzentrationswerts eines Analyten in einem Probenstrom, welcher den Analyten und ein Lösungsmittel aufweist, insbesondere nach dem erfindungsgemäßen Verfahren, wobei die Vorrichtung nicht Teil der anspruchsgemässen Erfindung ist, umfasst:

> Ein erstes Coriolis-Massedurchflussmessgerät, das eingerichtet ist zum Regeln eines Lösungsmittelstroms in Abhängigkeit vom Probenstrom zum Verdünnen des Probenstroms zu einem Messstrom;
> ein zweites Coriolis-Massedurchflussmessgerät das eingerichtet ist zum Ermitteln eines Massedurchflussmesswerts des Messstroms;
> ein Dichtemessgerät zum Ermitteln eines Dichtemesswerts des Messstroms; und
> eine Rechen- und Betriebseinheit die Eingerichtet ist zum Berechnen der Konzentration des Analyten in dem Probenstrom.

Die Vorrichtung umfasst einen Massedurchflussregler zum Regeln des Lösungsmittelstroms, wobei der Massedurchflussregler dazu eingerichtet ist, den Lösungsmittelstrom auf einen Sollwert zu regeln, der vom Massedurchflussmesswert des Messstroms abhängt, wobei das erste Coriolis-Massedurchflussmessgerät dazu eingerichtet ist, dem Massedurchflussregler einen Istwert des Lösungsmittelstroms bereitzustellen.

Die Erfindung wird nun anhand eines in den Zeichnungen dargestellten Ausführungsbeispiels näher erläutert.

Es zeigt:

Fig. 1: Eine schematische Darstellung eines Ausführungsbeispiels einer erfindungsgemäßen Vorrichtung zur Durchführung des erfindungsgemäßen Verfahrens;

Fig. 2: Ein Flussdiagramm eines Ausführungsbeispiels des Erfindungsgemäßen Verfahrens; und

Fig. 3: Messdaten zur Messwertstabilität des erfindungsgemäßen Verfahrens.

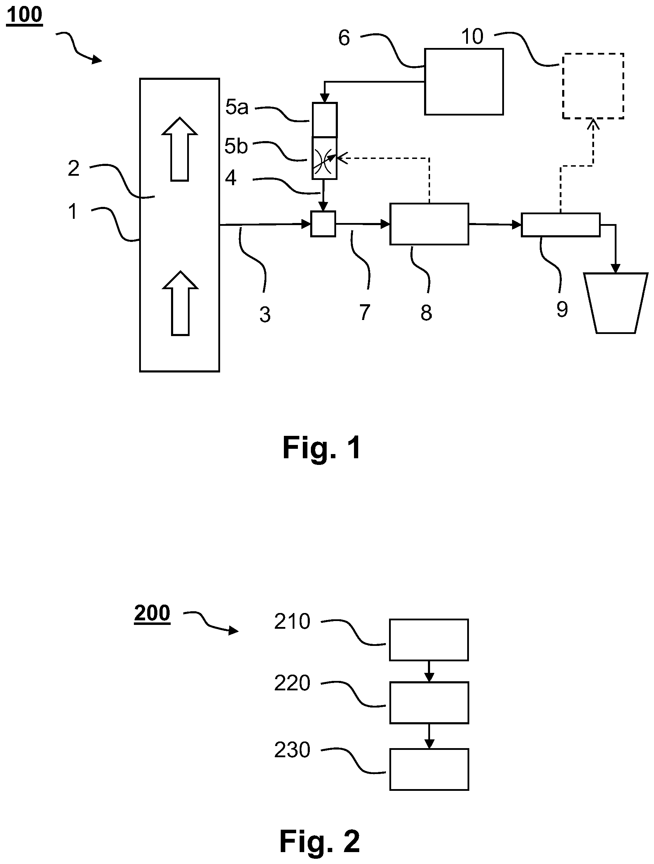

Die in Fig 1 dargestellte Vorrichtung 100 umfasst eine Rohrleitung 100 in der ein Massestrom 2 eines Prozessmediums, beispielsweise einer Sole fließt. Das Prozessmedium enthält einen Analyten in einem Lösungsmittel, im Beispiel also Kochsalz in Wasser, wobei die Konzentration ($X_3$) des Analyten zu bestimmen ist. Für die Prozessführung ist eine hohe Konzentration angestrebt, im Idealfall die Sättigungskonzentration, die durch eine Messung zu verifizieren ist. Dabei kann jedoch der Analyt ausfallen und dadurch die Messung verfälschen und im Extremfall die Messanordnung verstopfen. Zur Konzentrationsbestimmung wird daher erfindungsgemäß ein Probenstrom 3, der beispielsweise weniger als 0.1% des Massestroms des Prozessmediums beträgt, abgezweigt und durch Zufuhr eines Lösungsmittelstroms 7 aus einem Reservoir 6 in einem konstanten Verhältnis verdünnt, um einen Messstrom 7 zu präparieren, dessen Analytkonzentration deutlich unterhalb der Sättigungskonzentration liegt.

Das Reservoir kann im einfachsten Fall eine Wasserleitung umfassen, wobei der statische Druck des Lösungsmittelstroms 4 höher sein sollte als jener des Probenstroms 3.

Um ein konstantes Verdünnungsverhältnis zu erzielen, wird ein Massedurchflussratenmesswert ($\dot{m}_7$) des Messstroms 7 mit einem Coriolis Massedurchflussmessgerät 8 bestimmt. Der Massedurchflussratenmesswert ($\dot{m}_7$) wird vorzugsweise einem Massendurchfussregler (Flow-Controller) 5b mit einer einstellbaren Drossel zugeführt, der weiterhin einen Istwert des Lösungsmittelstroms 7 von einem vorgeschalteten Coriolis-Massedurchflussmessgerät 5a empfängt, und dazu eingerichtet ist, mittels der Drossel einen Massedurchflussratenmesswert ($\dot{m}_4$) des Lösungsmittelstroms 4 auf einen konstanten Anteil R des Massendurchflussratenmesswerts ($\dot{m}_7$) des Messstroms einzuregeln, also:

$$\dot{m}_4 = \dot{m}_7 \cdot R = (\dot{m}_3 + \dot{m}_4) \cdot R$$

Damit gilt für das Verhältnis der Massedurchflussrate ($\dot{m}_3$) des Probenmassenstroms 3 und der Massedurchflussrate ($\dot{m}_4$) des Lösungsmittelstroms 4:

$$\dot{m}_3 = \dot{m}_4 \frac{1-R}{R}$$

Die Konzentration des Analyten $X_3$ im Probenstrom 3 kann daher anhand der Konzentration $X_7$ des Analyten im Messstrom 7 bestimmt werden gemäß:

$$x_3 = x_7 \cdot \frac{\dot{m}_4 \frac{1-R}{R} + \dot{m}_4}{\dot{m}_4 \frac{1-R}{R}} = x_7 \cdot \frac{1}{1-R}$$

Zur Konzentrationsbestimmung wird ein Dichtemesswert des Messstroms 7 entweder mit dem Coriolis-Massedurchflussmessgerätes 8 oder einem anschließend angeordneten, vibronischen mikromechanischen Dichtemesser 9 ermittelt, und ein Temperaturmesswert des Messstroms 7 wird erfasst, wobei der Temperatursensor in den Dichtemesser 9 integriert ist. Der Messstromkonzentrationswert $X_7$ wird als Funktion des Dichtemesswerts und des Temperaturmesswerts berechnet wird. Nach dem Erfassen des Dichtemesswerts $\rho$ des Messstroms 7 wird der Messstrom verworfen. Die Berechnung des Messstromkonzentrationswert $X_7$ bzw. des Probenstromkonzentrationswert $X_3$ kann durch eine integrierte Recheneinheit des Dichtemessers 9 oder durch eine ggf. vorhandene separate Recheneinheit 10 durchgeführt werden, der die erforderlichen Messwerte für Dichte und Temperatur zugeführt werden.

Es ist nicht zwingend erforderlich, dass auf ein konstantes Verdünnungsverhältnis R geregelt wird. Es ist jedoch erforderlich, dass das Verdünnungsverhältnis exakt bekannt ist. Dazu reicht es aus, wenn das erste Coriolis-Massedurchflussmessgerät 5a zum Erfassen des Lösungsmittelstroms und das zweite Coriolis-Massedurchflussmessgerät 8 zum Erfassen des Messstroms 7 vorhanden sind. Ein ungeregeltes Verdünnungsverhältnis R in der richtigen Größenordnung kann mit einer konstanten oder einstellbaren Drossel im Lösumgsmittelstrom vorgegeben werden.

Insbesondere der Einsatz eines mikromechanischen Dichtemessers und Coriolis-Massedurchflussmessgeräten mit Nennweiten von 1 mm und weniger ermöglicht die kontinuierliche Konzentrationsbestimmung mit minimalen Probenmengen, so dass das Verwerfen des Messstroms ohne Weiteres zu vertreten ist. So sind die erforderlichen Messgenauigkeiten für die Massedurchflussraten bei DN1 ohne Weiteres mit Durchflussraten von 0.6 kg/h bis 6 kg/h zu erzielen. Bei DN0.4 reichen Durchflussraten von 0.06 kg/h bis 0.6kg/h aus.

Im vorliegenden Beispiel wird die Vorrichtung für die Konzentrationsbestimmung einer Sole beispielsweise mit R = 20% betrieben. Damit gilt für die Konzentration des Kochsalzes im Probenstrom:

$$x_{3,NaCl} = x_{7,NaCl} \cdot 1.25$$

Die Konzentration des Analyten im Messstrom beträgt vier Fünftel der Konzentration im Probenstrom, und weist damit einen ausreichenden Abstand von der Sättigungskonzentration auf, so dass ein Ausfallen des Analyten (hier Kochsalz) zuverlässig verhindert ist.

Zusammenfassend seien noch einmal unter Verweis auf Fig. 2 die wesentlichen Schritte des erfindungsmäßen Verfahrens genannt: Das Verfahren (200) zum Bestimmen eines Probenstromkonzentrationswerts (X$_3$) eines Analyten in einem Probenstrom, welcher den Analyten und ein Lösungsmittel aufweist, beginnt mit dem Präparieren (210) eines Messstromes durch Verdünnen des Probenstromes mit einem Lösungsmittelstrom. Auf diese Weise wird sichergestellt, dass die Konzentration des Analyten im Messstrom deutlich unterhalb der Sättigungskonzentration des Analyten liegt, und Ausfallen des Analyten die Messung nicht verfälschen kann. Im nächsten Schritt erfolgt das Ermitteln (220) eines Messstromzentrationswerts (X$_7$) des Analyten in dem Messstrom, was hier im Wesentlichen auf Basis einer Dichtemessung geschieht. Schließlich erfolgt das Ermitteln (230) des Probenstromkonzentrationswerts (X$_3$) des Analyten in dem Probenstrom durch Berechnung in Abhängigkeit vom Messstromkonzentrationswert (X$_7$) und dem Verdünnungsverhältnis (R) zwischen Probenstrom (3) und Messstrom (7).

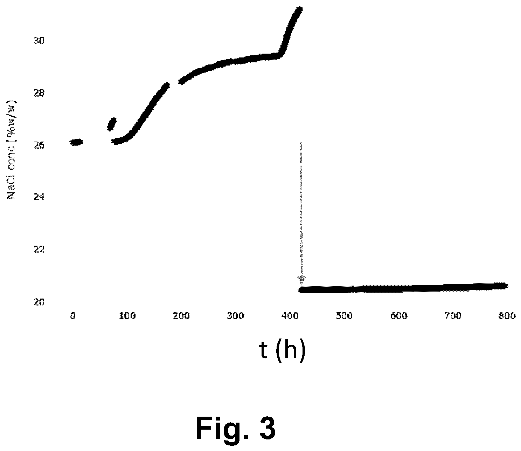

Fig. 3 zeigt bis t = 420 h Daten von Konzentrationsmessungen an einem unverdünnten Probenstrom mit etwa 26.1 Masse-% Salz auf Basis von Dichtemessungen an einer Sole mit einer konstanten Sättigungskonzentration. Hier wird scheinbar ein stetiger Zuwachs der Konzentration gemessenen, da sich Salz am Oszillator absetzt und insoweit einen Massezuwachs bewirkt, der die Messung verfälscht. Ab 420 Stunden erfolgte dagegen eine Verdünnung mit R = 22%. Die Messdaten zeigen die Konzentration des Salzes im Messstrom bei konstant 20,4 Masse-%. Die Umrechnung gemäß

$$x_3 = x_7 \cdot \frac{1}{1-R}$$

ergibt eine Konzentration von 26,15 Masse-% für den unverdünnten Probenstrom. Das ist hinsichtlich Messgenauigkeit und Messwertstabilität eine deutliche Verbesserung gegenüber dem Stand der Technik.

**Patentansprüche**

1.   Verfahren (200) zum Bestimmen eines Probenstromkonzentrationswerts (X$_3$) eines Analyten in einem Probenstrom (3), welcher den Analyten und ein Lösungsmittel aufweist, wobei das Verfahren folgende Schritte umfasst:

Präparieren (210) eines Messstromes (7) durch Verdünnen des Probenstromes (3) mit einem Lösungsmittelstrom (4);
Ermitteln (220) eines Messstromzentrationswerts (X$_7$) des Analyten in dem Messstrom (7); und
Ermitteln (230) des Probenstromkonzentrationswerts (X$_3$) des Analyten in dem Probenstrom, in Abhängigkeit des Messstromkonzentrationswerts (X$_7$) und eines Verdünnungsverhältnisses (R) von Probenstrom (3) und Messstrom (7),
wobei der Probenstrom (3) eine Konzentration des Analyten aufweist, welche eine Sättigungskonzentration des Analyten in dem Lösungsmittel um nicht mehr als 4 % der Sättigungskonzentration und insbesondere nicht mehr als 2 % der Sättigungskonzentration unterschreitet, und wobei der Messstrom (7) eine Konzentration des Analyten aufweist, welche eine Sättigungskonzentration des Analyten in dem Lösungsmittel um nicht weniger als 8 % der Sättigungskonzentration und insbesondere nicht weniger als 12 % der Sättigungskonzentration unterschreitet.

2.   Verfahren nach Anspruch 1, wobei das Ermitteln (220) des Messstromkonzentrationswerts (X$_7$) das Erfassen einer Messgröße umfasst, welche von der Konzentration des Messstroms in dem Analyten abhängt.

3.   Verfahren nach Anspruch 2, wobei die Messgröße die Dichte des Messstroms (7) umfasst.

4.   Verfahren nach einem der vorhergehenden Ansprüche, wobei das Verdünnungsverhältnis (R) auf Basis eines Massedurchflussratenmesswerts ($\dot{m}_4$) des Lösungsmittelstroms (4) und eines Massedurchflussratenmesswerts ($\dot{m}_7$) des Messstroms (7) ermittelt wird.

5.   Verfahren nach Anspruch einem der vorhergehenden Ansprüche, wobei das Verdünnungsverhältnis (R) auf einen konstanten Wert geregelt wird.

6.   Verfahren nach Anspruch 5, wobei das Verdünnungsverhältnis geregelt wird, indem der Massdurchflussratenmesswert ($\dot{m}_4$) des Lösungsmittelstroms (4) auf ein konstantes Verhältnis zum Massendurchflussratenmesswert ($\dot{m}_7$) des Messstroms (7) geregelt wird, wobei das Verhältnis nicht mehr 1/2, beispielsweise nicht mehr als 1/4, und insbesondere nicht mehr als 1/5 beträgt.

7. Verfahren nach Anspruch einem der vorhergehenden Ansprüche, wobei der Analyt ein Salz umfasst, insbesondere NaCl.

8. Verfahren nach einem der vorhergehenden Ansprüche wobei das Lösungsmittel ein wässriges Medium umfasst, insbesondere Wasser.

9. Verfahren nach Anspruch 3, wobei ein Dichtemesswert für die Dichte des Messstroms (7) mittels eines vibronischen Sensors (9) auf Basis mindestens einer Schwingfrequenz des vibronischen Sensors (9) ermittelt wird.

10. Verfahren nach einem der Ansprüche 2 und 4 und ggf. davon abhängigen vorhergehenden Ansprüchen, wobei der Messstrom (7) nach Erfassen der Messgröße und Bestimmen des Massendurchflussratenmesswerts ($\dot{m}_7$) des Messstroms verworfen wird.

11. Verfahren nach einem der vorhergehenden Ansprüche, wobei der Probenstrom (7) einem Prozessmedienstrom (2) entnommen ist, dessen Massedurchflussrate nicht weniger als das Hundertfache insbesondere nicht weniger als das Tausendfache einer Massedurchflussrate des Probenstroms ($\dot{m}_3$) beträgt, wobei der Probenstromkonzentrationswert als Prozessmedienkonzentrationswert ($X_3$) ausgegeben wird.

**Claims**

1. Method (200) for determining a sample stream concentration value ($X_3$) of an analyte in a sample stream (3) comprising the analyte and a solvent, the method comprising the steps of:

   preparing (210) a measuring stream (7) by diluting the sample stream (3) with a solvent stream (4);
   determining (220) a measuring stream centration value ($X_7$) of the analyte in the measuring stream (7); and
   determining (230) the sample stream concentration value ($X_3$) of the analyte in the sample stream as a function of the measuring stream concentration value ($X_7$) and a dilution ratio (R) of sample stream (3) and measuring stream (7), wherein the sample stream (3) has a concentration of the analyte which is not more than 4% of the saturation concentration and in particular not more than 2% of the saturation concentration below a saturation concentration of the analyte in the solvent, and

wherein the measuring current (7) has a concentration of the analyte which falls below a saturation concentration of the analyte in the solvent by not less than 8% of the saturation concentration and in particular not less than 12% of the saturation concentration.

2. Method according to claim 1, wherein determining (220) the measuring stream concentration value ($X_7$) comprises sensing a measure that depends on the concentration of the measuring stream in the analyte.

3. Method according to claim 2, wherein the measure comprises the density of the measuring stream (7).

4. Method according to one of the preceding claims, wherein the dilution ratio (R) is determined on the basis of a measured mass flow rate ($\dot{m}_4$) of the solvent stream (4) and a measured mass flow rate ($\dot{m}_7$) of the measuring stream (7).

5. Method according to one of the preceding claims, wherein the dilution ratio (R) is controlled to a constant value.

6. Method according to claim 5, wherein the dilution ratio is controlled by controlling the mass flow rate measurement value ($\dot{m}_4$) of the solvent stream (4) to be in a constant ratio to the mass flow rate measurement value ($\dot{m}_7$) of the measuring stream (7), wherein the ratio is no longer 1/2, for example no longer than 1/4, and in particular no longer than 1/5.

7. Method according to one of the preceding claims, wherein the analyte comprises a salt, in particular NaCl.

8. Method according to one of the preceding claims, wherein the solvent comprises an aqueous medium, in particular water.

9. Method according to claim 3, wherein a measured density value for the density of the measuring stream (7) is determined by means of a vibronic based on at least one vibration frequency of the vibronic sensor (9).

10. Method according to one of the claims 2 and 4 and, if applicable, dependent preceding claims, wherein the measuring stream (7) is discarded after detecting the measured variable and determining the mass flow rate measured value ($\dot{m}_7$) of the measuring stream.

11. Method according to one of the preceding claims, wherein the sample flow (7) is taken from a process media flow (2) whose mass flow rate is not less than one hundred times, in particular not less than one thousand times, a mass flow rate of the sample flow ($\dot{m}_3$), wherein the sample flow concentration value is output as a process media concentration value ($X_3$).

## Revendications

1. Procédé (200) pour déterminer une valeur de concentration d'un échantillon ($X_3$) d'un analyte dans un échantillon (3) qui contient l'analyte et un solvant, le procédé comprenant les étapes suivantes :

   préparation (210) d'un échantillon de mesure (7) par dilution de l'échantillon (3) avec un échantillon de solvant (4) ;
   détermination (220) d'une valeur de concentration du courant de mesure ($X_7$) de l'analyte dans le courant de mesure (7) ; et
   détermination (230) de la valeur de concentration du courant d'échantillon ($X_3$) de l'analyte dans le courant d'échantillon, en fonction de la valeur de concentration du courant de mesure ($X_7$) et d'un rapport de dilution (R) du courant d'échantillon (3) et du courant de mesure (7),
   le flux d'échantillon (3) présentant une concentration de l'analyte qui ne descend pas en dessous d'une concentration de saturation de l'analyte dans le solvant de plus de 4 % de la concentration de saturation et en particulier de plus de 2 % de la concentration de saturation, et le courant de mesure (7) présentant une concentration de l'analyte qui ne dépasse pas une concentration de saturation de l'analyte dans le solvant de moins de 8 % de la concentration de saturation et en particulier de moins de 12 % de la concentration de saturation.

2. Procédé selon la revendication 1, dans lequel la détermination (220) de la valeur de concentration du courant de mesure ($X_7$) comprend l'enregistrement d'une grandeur de mesure qui dépend de la concentration de l'analyte dans le courant de mesure.

3. Procédé selon la revendication 2, dans lequel la grandeur de mesure comprend la densité du courant de mesure (7).

4. Procédé selon l'une des revendications précédentes, dans lequel le rapport de dilution (R) est déterminé sur la base d'une valeur de mesure du débit massique ($\dot{m}_4$) du flux de solvant (4) et d'une valeur de mesure du débit massique ($\dot{m}_7$) du flux de mesure (7).

5. Procédé selon l'une des revendications précédentes, dans lequel le rapport de dilution (R) est réglé à une valeur constante.

6. Procédé selon la revendication 5, dans lequel le rapport de dilution est régulé en régulant la valeur de mesure du débit massique ($\dot{m}_4$) du flux de solvant (4) à un rapport constant par rapport à la valeur de mesure du débit massique ($\dot{m}_7$) du flux de mesure (7), le rapport n'étant plus de 1/2, par exemple pas plus de 1/4, et en particulier pas plus de 1/5.

7. Procédé selon l'une des revendications précédentes, dans lequel l'analyte comprend un sel, en particulier du NaCl.

8. Procédé selon l'une des revendications précédentes, dans lequel le solvant comprend un milieu aqueux, en particulier de l'eau.

9. Procédé selon la revendication 3, dans lequel une valeur de mesure de la densité du courant de mesure (7) est déterminée au moyen d'un vibronique basé sur au moins une fréquence d'oscillation du capteur vibronique (9).

10. Procédé selon l'une des revendications 2 et 4 et, le cas échéant, selon des revendications précédentes qui en dépendent, dans lequel le courant de mesure (7) est rejeté après détection de la grandeur de mesure et détermination de la valeur de mesure du débit massique ($\dot{m}_7$) du courant de mesure.

11. Procédé selon l'une des revendications précédentes, dans lequel le courant d'échantillon (7) est prélevé d'un courant de fluide de processus (2) dont le débit massique n'est pas inférieur à cent fois, en particulier pas inférieur à mille fois, un débit massique du courant d'échantillon ($\dot{m}_3$), la valeur de concentration du courant d'échantillon étant émise en tant que valeur de concentration du fluide de processus ($X_3$).

**100**

6  10

5a

5b

2

1

4

3  7  8  9

**Fig. 1**

**200**

210

220

230

**Fig. 2**

Fig. 3